# EUROPÄISCHE PATENTSCHRIFT

(11) **EP 1 796 555 B1**
(45) Veröffentlichungstag und Bekanntmachung des Hinweises auf die Patenterteilung: **26.05.2010**
(21) Anmeldenummer: 05784767.5
(22) Anmeldetag: 22.09.2005
(51) Int. Cl.: A61B 17/128

(54) **APPLIKATIONSVORRICHTUNG FÜR KOMPRESSIONSMANSCHETTEN**
APPLICATION DEVICE FOR COMPRESSIVE CUFFS
DISPOSITIF DE POSE DE MANCHONS DE COMPRESSION

(30) Priorität: 27.09.2004 DE 102004046840
(43) Veröffentlichungstag der Anmeldung: 20.06.2007
(73) Patentinhaber: Karlsruher Institut für Technologie, 76131 Karlsruhe (DE)
(72) Erfinder: KLEIN, Marco, 76774 Leimersheim (DE); STAIER, Christian, 74889 Sinsheim (DE); VOGEL, Bernd, 76199 Karlsruhe (DE)
(74) Vertreter: Weddigen, Andreas
(86) Internationale Anmeldenummer: PCT/EP2005/010250
(87) Internationale Veröffentlichungsnummer: WO 2006/034816

(56) Entgegenhaltungen:
- US-A- 5 211 649
- US-A1- 2002 022 837
- US-B1- 6 648 911

## Beschreibung

Die Erfindung betrifft eine Applikationsvorrichtung für Kompressionsmanschetten, wie sie z.B. in der DE 103 55 986 im Prinzip beschrieben sind, zur lokalen Ummantelung eines Blutgefäßes beispielsweise zur Stützung einer körpereigenen Venenklappe gemäß des Anspruchs 1.

Gewebeschwächen im menschlichen Köper, insbesondere bei Blutgefäßen führen im zunehmenden Alter zu signifikanten Gefäßerweiterungen. Dieser Effekt verstärkt sich insbesondere dann, wenn die körpereigenen Ventilsysteme im Blutkreislauf so weit gedehnt werden, dass sie nicht mehr als unidirektionale Ventile (Rückschlagventile oder Strömungsventile) funktionieren, sodass es zu Refluxerscheinungen und damit zu einer zusätzlichen inneren Druckbelastung einzelner Blutgefäße kommt.

Untherapiert fördert dieser Effekt massiv die Bildung so genannter Krampfadern bevorzugt im Beinbereich (Varikose). Die Bildung beginnt zunächst mit einer Gefäßerweiterung insbesondere an schwachen oder nachgiebigen Gewebebereichen als Gefäßwandungen. In Folge dessen können sich auch die körpereigenen Venenklappen beispielsweise im Beckenbereich dehnen, wobei diese mit fortschreitendem Befund meist versagen. Schließlich kommt es, unterstützt durch die Schwerkraft, zu einem Rückstau des Blutes und damit zu einer Druckbelastung und weiteren Aufdehnungen im Venenbereich der Beine. Die Aufdehnung (Gefäßerweiterung, Ditalieren) der Venen führen zunächst zu der Bildung von Krampfadern, in einem fortgeschrittenen Stadium zum Befund eines offenen Beins.

Für eine nachhaltige oder vorbeugende Behandlung von Krampfadern ist dagegen die Aufrechterhaltung oder Wiederherstellung der Funktionsfähigkeit der Venen und der Venenklappen von besonderer Bedeutung. Idealerweise muss eine Vermeidung einer Dilatation von Venen oder Venenklappen bereits im dilatierten Gewebe, vorzugsweise durch eine Verstärkung des Gewebes, angreifen.

Aus der US 5.500.014 sind mehrere Ausführungsformen einer Stützmanschette für ein Blutgefäß bekannt, wobei allen eine rohrförmige Gestaltung gemein ist. Folglich muss das Blutgefäß bei einer Applikation in die Manschette eingefädelt und dabei zuvor durchschnitten werden. Die Stützmanschetten sind nicht für ein schnelles Verwachsen mit dem Gewebe des Blutgefäßes ausgelegt.

Dieser Nachteil wird, wie in der DE 103 55 986 beschrieben, durch den Einsatz einer verbesserten Kompressionsmanschette, im Wesentlichen bestehend aus einer biegeweichen Matte aus einem biokompatiblen Material (z.B. Titan, Nickel-Titan-Legierungen) vermieden. Mit dieser Matte wird ein Blutgefäß lokal begrenzt, vorzugsweise im Bereich einer Gewebeschwächung oder eines Venenklappenbereichs, ummantelt. Ein Durchschneiden der Blutgefäße ist dabei nicht mehr erforderlich. In einer bevorzugten Ausführungsform genügt allein eine superelastische Vorspannung der Matte zur zuverlässigen Fixierung auf dem Blutgefäß.

Eine Applikationsvorrichtung ist im Dokument US 2002/0022 837 offenbart und zeigt die Merkmale des einführenden Teils von Anspruch 1.

Ausgehend davon hat die Erfindung zur Aufgabe, eine Applikationsvorrichtung für die zuletzt genannten superelastischen Kompressionsmanschetten vorzuschlagen, welche einen chirurgischen Eingriff der Applikation unter weitestgehender Schonung des Patienten ermöglicht.

Zur Lösung der Aufgabe wird im Rahmen einer Erfindung eine Applikationsvorrichtung mit den Merkmalen aus Patentanspruch 1 vorgeschlagen. Weitere, vorteilhafte und die Erfindung weiterbildende Merkmale sind in den Unteransprüchen wiedergegeben.

Die Applikationsvorrichtung dient der lokalen Ummantelung eines Blutgefäßes mit einer Kompressionsmanschette, wobei eine elastisch aufgebogene Kompressionsmanschette auf einem Träger zwangsgeführt auf ein über eine Führung positioniertes Blutgefäß aufgeschoben wird und sich dort elastisch auf der Wandung des Blutgefäßes anschmiegt. Der Aufschiebeprczess erfolgt über ein Schiebeelement. Die Schieberichtung kann dabei axial oder radial zur Erstreckung der Kompressionsmanschette auf dem Träger sein, wobei ein axiales Aufschieben einer aufgebogenen Kompressionsmanschette vorzugsweise im spitzen Winkel zum Blutgefäß erfolgt.

Zu diesem Zweck umfasst die Applikationsvorrichtung ein Endstück, umfassend Führung oder Fixierung für das Blutgefäß während des Eingriffs wie z.B. ein längs geschlitztes, beidseitig offenes Rohrstück oder einen U-förmig gebogenen Blechstreifen sowie einen Träger für eine elastisch aufgebogene Kompressionsmanschette. Dabei kann die Führung grundsätzlich auch die Funktion des Trägers mit übernehmen. Das Blutgefäß wird in die Führung, d.h. über den Längsschlitz oder die offene U-Flanke in das Rohstück eingelegt und durch dieses relativ zum Träger ausgerichtet.

Die Führung sollte dem Blutgefäß gestatten, möglichen Belastungen beim Aufschiebeprozess nachzugeben, d. h. das Blutgefäß sollte sich in der Führung drehen oder axial verschieben lassen. Das genannte Rohrstück ist vorzugsweise gerade oder leicht gekrümmt, sodass sich das Blutgefäß ohne größeren Widerstand axial oder um die Rohrstückachse im Rohrstück verschiebbar bzw. drehbar ist.

Proximalseitig ist in Verlängerung des Trägers ein Führungselement angebracht, mit dem der Träger während eines Applikationseingriffs im Patientenkörper positionierbar ist. Ferner umfasst die Applikationsvorrichtung ein Schiebeelement zum bevorzugt distalseitigen Herunterschieben der Komponente über das Blutgefäß.

Das Endstück ist vorzugsweise V-förmig, d.h. Träger und Rohrstück bilden die vorzugsweise distalseitig zusammenlaufenden Flanken des V-förmigen Endstücks. Sie spannen sich proximalseitig einen spitzen Winkel auf und durchdringen sich gegenseitig im zusammenlaufenden Bereich, wobei das Rohrstück in seinen vorzugsweise zylindrischen Innenabmessungen unverändert verbleibt. Dabei ist das offene Rohrstückende im zusammenlaufenden Bereich innerhalb des Endquerschnitts des Trägers positioniert. Es bietet sich an, für den Träger mit einem größeren Querschnitt als das Rohrstück zu wählen, damit bei dem vorgenannten Herunterschieben der Kompressionsmanschette ein weiteres, temporäres Aufweiten dieser über das Rohrstück im Durchdringungsbereich möglichst vermieden oder zumindest geometrisch begrenzt wird. Dabei wird die Kompressionsmanschette über in einem spitzen Winkel α mit dem Träger zusammenlaufende Rohrstück kurzzeitig zusätzlich aufgeweitet und damit über das Blutgefäß aufgeschoben.

Die Erfindung sowie einzelne Details oder Teilaspekte dieser werden an konkreten Ausführungsbeispielen mit den folgenden Figuren näher erläutert. Es zeigen
Fig. 1 eine Applikationsvorrichtung einer ersten Ausführungsform mit einer auf den Träger aufgesetzte zylinderförmige Kompressionsmanschette,
Fig. 2 das Endstück der ersten Ausführungsform mit aufgesetzter Kompressionsmanschette im Detail,
Fig. 3 a und b das Endstück gemäß Fig. 2 in zwei weiteren Ansichten,
Fig. 4 eine zweite Ausführungsform, welche sich von der ersten Ausführungsform im Wesentlich durch eine andere Gestaltung des Schiebeelementes unterscheidet,
Fig. 5 eine dritte Ausführungsform mit einem Endstück, umfassend einen U-förmig gebogenen Blechstreifen sowie
Fig. 6 a und b das Endstück der dritten Ausführungsform, umfassend einen U-förmig gebogenen Blechstreifen ohne und mit aufgeschobener Kompressionsmanschette.

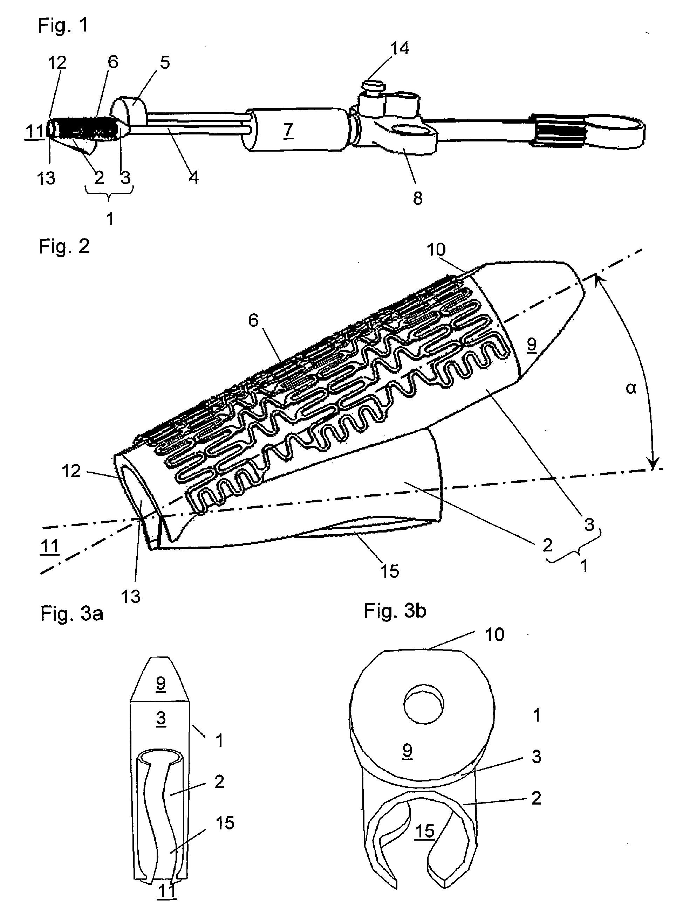

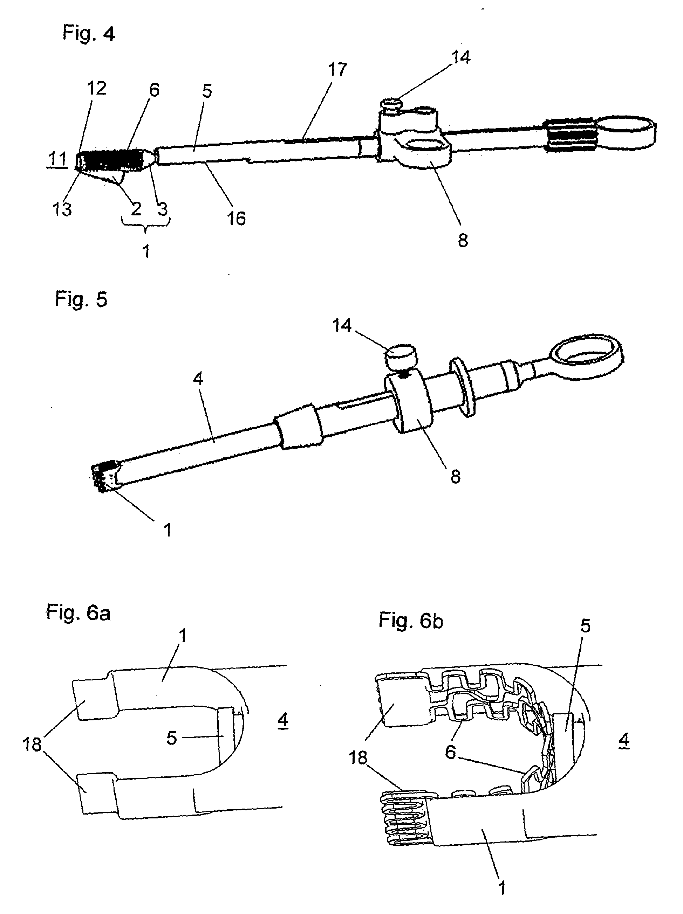

Die ersten beiden Ausführungsformen (Fig. 1 bis 4) umfassen im Wesentlichen das vorgenannte V-förmige Endstück 1, bestehend aus einem Rohrstück 2 und einem Träger 3, einem Führungselement 4 und einem Schiebeelement 5 zum distalen Herunterschieben einer auf dem Träger aufgesetzte, vorzugsweise zylinderförmige Kompressionsmanschette 6, wie sie im Prinzip in der DE 103 55 986 beschrieben wurde. Weiterhin umfasst die dargestellte Ausführungsform einen Führungsblock 7 zur parallelen Führung sowie ein Griffstück 8 mit einer Mechanik zu gegenläufigen Bewegung des Führungselementes 4 und des Schiebeelements 5. In der perspektivischen Darstellung der zweiten Ausführungsform gem. Fig. 4 befindet sich das Führungselement 4 nicht sichtbar im Bereich des Schiebelements 5.

Bei der vorgenannten gegenläufigen Bewegung kann entweder der Führungs- oder das Schiebeelement durch geeignete Mittel, in Fig. 1 und 4 als Drehknopf 14 für eine Arretierung ausgeführt, fest im Griffstück fixiert sein. Ein Herunterschieben einer Kompressionsmanschette auf ein Blutgefäß erfolgt besonders gewebeschonend, wenn die Kompressionsmanschette möglichst keine axialen Relativbewegung zum Blutgefäß ausführt, d.h. das Endstück unter der Kompressionsmanschette axial auf dem Blutgefäß hinweg gezogen wird.

Das Schiebeelement 5 der ersten Ausführungsform ist als kompakte Komponente auf einer Schiebestange aufgebracht und belastet die Kompressionsmanschette während des Aufschiebeprozess lediglich auf dem oberen Bereich oberhalb der Nut 10 (vgl. Fig. 2 und 3 a) nur punktuell. Die Nut 10 dient der Führung der vorgenannten Komponente und vermeidet insbesondere ein Abrutschen der Komponente von der Stirnseite auf die Oberfläche der Kompressionsmanschette auf dem Träger. Diese sichernde Wirkung kann durch Strukturen auf der Stirnseite des Schiebeelements noch verstärkt werden.

Dagegen umfasst das Schiebeelement gemäß der zweiten Ausführungsform (Fig. 4) ein Schiebelement in der Form eines Rohrs, welches distalseitig durch den Träger geführt auf einen Großteil der Kompressionsmanschettenstirnseite flächig angreift. Das vorgenannte Ab- oder Wegrutschen (erste Ausführungsform) wird durch eine geringe Spielpassung zwischen Rohrinnenfläche und Träger realisiert. Eine Nut der vorgenannten Art ist hier nicht unbedingt erforderlich.

Diese kompaktere Bauform des Schiebelements dieser zweiten Ausführungsform hat jedoch die Einschränkung, dass sie unmodifiziert nicht über den Durchdringungsbereich zwischen Rohrstück und Träger überschiebbar ist. Aus diesem Grund bietet es sich an, das rohrförmige Schiebeelement radial dehnbar, d.h. einfach oder mehrfach geschlitzt zu gestalten, wobei alternativ oder in Ergänzung die bei einer Aufweitung besonders verformten Bereiche konstruktiv oder stofflich elastisch nachgiebiger gestaltet werden können, beispielsweise durch gezielte Materialschwächung oder durch eine Verwendung von Elastomeren. Fig. 4 beispielsweise offenbart ein Schiebeelement 5 in der Form eines Rohres, welches im distalen Bereich einen breiten Aussparung 16 aufweist. Eine ebenfalls offenbarte Führungsnut 17 dient als zusätzliche Führung im Griffstück, beispielsweise über ein Stiftende, welches mit dem Drehknopf 14 verbunden ist.

Das Endstück der ersten Ausführungsform ist in den Figuren 2 und 3 a und b im Detail wiedergegeben.

Fig. 2 gibt besonders die V-förmige Gestalt des Endstücks sowie die Durchdringung von Träger 3 und Rohrstück 2 wieder. Fig. 3 a und b zeigt das Endstück von unten bzw. proximalseitig, wobei der Längsschlitz 15 im Rohrstück gut erkennbar ist. Durch den Längsschlitz wird das Blutgefäß vor einer Applikation eingeführt. Vorzugsweise erstreckt sich der Längsschlitzschlitz in einer Wellenlinie auf der Mantelfläche des Rohrstücks. Abschnitte des Längsschlitzes verlaufen windschief zur Rohrachse. Der Träger ist unter der aufgespannten Kompressionsmanschette 6 zylinderförmig gestaltet und weist proximalseitig einen konischen Bereich 9 als Applikationshilfe (axiales Aufschieben) einer Kompressionsmanschette auf den Träger. Vorzugsweise auf der Oberseite des zylinderförmigen Bereichs des Trägers befindet sich zudem eine Nut 10 zur besseren Führung des in Fig. 1 dargestellten Schiebelements 5 auf dem Träger 3. Ferner überdeckt im distalen Bereich 11 der Endquerschnitt 12 des Trägers die distalseitigen Öffnungsquerschnitt 13 des Rohrstücks. Bei einer Applikation (Herunterschieben) befindet sich das Blutgefäß in vorteilhafter Weise bereits vollständig innerhalb der Kompressionsmanschette, wobei die elastische Vorspannung ein Anpressen der Kompressionsmanschette auf das Blutgefäß einleitet und den Applikationsvorgang beendet. Die Abwinkelung des V-förmigen Endstücks beträgt in einem Winkel α vorzugsweise zwischen 20 und 30°. Die Kompressionsmanschette steht dann unter einem Winkel von α = 20 bis 30 Grad zum Gefäß im Rohrstück.

Das Endstück der zweiten Ausführungsform entspricht im Wesentlichen des in Fig. 2 und 3 gezeigten, wobei aus vorgenannten Gründen die Nut 10 entfallen kann.

Das Aufschieben einer zylinderförmigen Kompressionsmanschette mit einem beispielhaften Durchmesser von ca. 5 mm erfolgt über das konische Endstück, wodurch sich die Kompressionsmanschette im Beispiel auf einen Durchmesser von ca. 10 mm aufdehnt.

Es bietet sich an und liegt grundsätzlich im Bereich der Erfindung, die Applikationsvorrichtung zweiteilig zu gestalten, wobei eine Baugruppe als Einwegkomponente das Endstück vorzugsweise mit bereits industriell aufgesetzter Kompressionsmanschette, und eine andere Baugruppe als Mehrwegteil das Griffstück umfasst. Bestimmte Teile, wie beispielsweise der Führungsblock, das Führungselement und/oder das Schiebeelement können je nach Auslegung zum Einweg- oder Mehrwegteil ganz oder jeweils zum Teil in der ersten oder zweiten Baugruppe integriert werden.

Eine dritte Ausführungsform zeigen die Figuren 5, 6a und 6b. Wie bei den ersten beiden Ausführungsbeispielen umfasst die Applikationsvorrichtung ein Griffstück 8 mit Drehknopf 14 zur Arretierung des Schiebeelements. Als Führungselement 4 dient ein Rohr, auf dessen distalen Ende das Endstück 1 aufgesetzt ist. Das Endstück umfasst im Wesentlichen einen U-förmig gebogenen Blechstreifen als Führung für das Blutgefäß und als Halterung für die Kompressionsmanschette 6 (vgl. Fig. 6b). Im Führungselement 4 ist das Schiebeelement 5 axial geführt (vgl. Fig. 6a und 6b), wobei das distale Ende des Schiebeelements zwischen den beiden Flanken 18 des U-förmig gebogenen Blechstreifens in das Innere des U-förmig gebogenen Blechstreifen hineinragt. Durch eine gegenläufige Axialbewegung zwischen Führungselement 4 und Schiebeelement 5 lässt sich das Schiebeelement 5 in distale Richtung verschieben, wobei es flächig mittig auf der in die beiden vorgenannten Flanken 18 eingesetzte und geführte Kompressionsmanschette 6 angreift, diese in distale Richtung vor sich herschiebt und vom Endstück über das nicht dargestellte, vorzugsweise orthogonal zur Vorrichtungserstreckung ausgerichtete Blutgefäß schiebt (vgl. Fig. 6b).

### Bezugszeichenliste

- 1: Endstück
- 2: Rohrstück
- 3: Träger
- 4: Führungselement
- 5: Schiebeelement
- 6: Kompressionsmanschette
- 7: Führungsblock
- 8: Griffstück
- 9: konischer Bereich
- 10: Nut
- 11: distaler Bereich
- 12: Endquerschnitt
- 13: Öffnungsquerschnitt
- 14: Drehknopf
- 15: Längsschlitz
- 16: Aussparung
- 17: Führungsnut
- 18: Flanken

## Patentansprüche

1. Applikationsvorrichtung für Kompressionsmanschetten zur lokalen Ummantelung eines Blutgefäßes, umfassend
einen Träger (3) zum Aufsetzen einer aufgebogenen Kompressionsmanschette (6) sowie
ein Schiebelement (5) zum Herunterschieben der Kompressionsmanschette vom Träger auf das Blutgefäß,
**dadurch gekennzeichnet, dass** die Applikationsvorrichtung eine Führung für das Blutgefäß enthält.

2. Applikationsvorrichtung nach Anspruch 1, wobei die Führung und der Träger zu einem V-förmigen Endstück (1) zusammengefasst sind, die Führung aus einem beidseitig offenen Rohrstück (2) mit einem Längsschlitz (15) zum Einbringen eines Blutgefäßes besteht, wobei der Träger und das Rohrstück als Flanken des V-förmigen Endstücks dienen, proximalseitig einen spitzen Winkel (α) aufspannen und sich distalseitig gegenseitig durchdringen sowie ein Führungselement in Verlängerung der trägerseitigen Flanke vorgesehen ist.

3. Applikationsvorrichtung nach Anspruch 2, wobei Abschnitte des Längsschlitzes windschief zu der Rohrstückachse verlaufen.

4. Applikationsvorrichtung nach Anspruch 1, 2 oder 3, wobei der Träger (3) einen zylinderförmigen Bereich als Aufsetzfläche der Kompressionsmanschette (6) aufweist, dessen Radius dem des Rohrstücks (2) übersteigt.

5. Applikationsvorrichtung nach einem der vorgenannten Ansprüche 2 bis 4, wobei der Träger proximalseitig einen konischen Bereich (9) zum Aufschieben der Kompressionsmanschette (6) aufweist.

6. Applikationsvorrichtung nach Anspruch 1, wobei die Führung und der Träger zu einem Endstück (1) in der Form eines U-förmig gebogenen Blechstreifen zusammengefasst sind, die Führung durch das Innere des U-förmig gebogenen Blechstreifen zum Einbringen eines Blutgefäßes und der Träger durch die beiden Flanken des U-förmig gebogenen Blechstreifen gebildet wird.

7. Applikationsvorrichtung nach einem der vorgenannten Ansprüche 2 bis 6, wobei das Endstück (1) Teil einer Einwegbaugruppe ist.

8. Applikationsvorrichtung nach Anspruch 7, wobei die Einwegbaugruppe ein bereits auf dem Endstück applizierte Kompressionsmanschette (6) umfasst.

## Claims

1. Application device for compression sleeves for local sheathing of a blood vessel, comprising
- a bracket (3) that holds a bent-open compression sleeve (6), and
- a slide element (5) that slides the compression sleeve off the bracket onto the blood vessel, the application device being provided with a guide element for the blood vessel.

2. Application device according to Claim 1, wherein the guide element (4) and the bracket (3) form a V-shaped end piece (1), the guide element consists of a tube section (2) that is open on both sides and is provided with a longitudinal slot (15) for receiving the blood vessel, the bracket and the tube section serve as the flanks of the V-shaped end piece and are arranged at an acute angle (α) on the proximal side while penetrating each other on the distal side, and a guide element is provided in the extension of the bracket-side flank.

3. Application device according to Claim 2, wherein sections of the longitudinal slot (15) extend skewed with respect to the axis of the tube section.

4. Application device according to Claim 1, 2 or 3, wherein the bracket (3) is provided with a cylindrical area for supporting the compression sleeve (6), the diameter of the cylindrical area exceeding that of the tube section (2).

5. Application device according to one of Claims 2 through 4, wherein the bracket (3) is provided on its proximal side with a conical area (9) for sliding the compression sleeve (6) onto the bracket (3).

6. Application device according to Claim 1, wherein the guide element (4) and the bracket (3) are combined into one end piece (1) in the form of a U-shaped bent metal strip, the guide element is formed by the interior of the U-shaped bent metal strip for reception of the blood vessel, and the bracket is formed by the two flanks of the U-shaped bent metal strip.

7. Application device according to one of Claims 2 through 6, wherein the end piece (1) is part of a one-way component.

8. Application device according to Claim 7, wherein the one-way component includes a compression sleeve (6) that is already disposed on the end piece (1).

## Revendications

1. Dispositif de pose de manchons de compression pour le revêtement local d'un vaisseau sanguin comportant un élément support (3) pour poser un manchon de compression (6) recourbé ainsi qu'un élément de glissement (5) pour faire descendre par glissement le manchon de compression de l'élément support sur le vaisseau sanguin,
**caractérisé en ce que**
le dispositif de pose comporte un élément de guidage pour le vaisseau sanguin.

2. Dispositif de pose selon la revendication 1,
dans lequel l'élément de guidage et l'élément support sont rassemblés en une pièce d'extrémité (1) en forme de V, l'élément de guidage étant constitué d'une pièce tubulaire (2) ouverte de part et d'autre comportant une fente longitudinale (15) pour l'introduction d'un vaisseau sanguin, l'élément support la pièce tubulaire définissant les branches de la pièce d'extrémité en forme de V, délimitant, un angle aiguë α côté proximal et pénétrant l'un dans l'autre côté distal, et un organe de guidage étant prévu dans le prolongement de la branche située côté support.

3. Dispositif de pose selon la revendication 2,
dans lequel des tronçons de la fente longitudinale s'étendent en étant voilés par rapport à l'axe de la pièce tubulaire.

4. Dispositif de pose selon la revendication 1, 2 ou 3,
dans lequel l'élément support (3) comporte une zone de forme de cylindre constituant la surface de pose du manchon de compression (6) et dont le rayon est supérieur à celui de la pièce tubulaire (2).

5. Dispositif de pose selon l'une des revendications 2 à 4,
dans lequel l'élément support comporte, côté proximal une zone conique (9) pour faire glisser le manchon de compression (6) en le poussant.

6. Dispositif de pose selon la revendication 1,
dans lequel l'élément de guidage et l'élément support sont rassemblés en une pièce d'extrémité (1) sous la forme d'une bande de tôle recourbée en forme de U, la pièce de guidage étant formée par la partie interne de cette bande de tôle recourbée en forme de U pour l'introduction d'un vaisseau sanguin et l'élément support étant formé par les deux branches de cette bande de tôle recourbée en forme de U.

7. Dispositif de pose selon l'une des revendications 2 à 6,
dans lequel la pièce d'extrémité (1) constitue une partie d'un élément jetable.

8. Dispositif de pose selon la revendication 7,
dans lequel l'élément jetable comporte un manchon de compression (6) déjà appliqué sur la pièce d'extrémité.
